# FASCICULE DE BREVET EUROPEEN

(11) **EP 0 988 279 B1**
(45) Date de publication et mention de la délivrance du brevet: **26.06.2002**
(21) Numéro de dépôt: 98928384.1
(22) Date de dépôt: 28.05.1998
(51) Int. Cl.: C07C 233/05, A61K 7/48

(54) **COMPOSITION COSMETIQUE COMPRENANT UN AMIDE ET NOUVEAUX AMIDES**
AMID ENTHALTENDE KOSMETISCHE ZUSAMMENSETZUNGEN UND NEUE AMIDE
COSMETIC COMPOSITION COMPRISING AN AMIDE AND NOVEL AMIDES

(30) Priorité: 11.06.1997 FR 9707241
(43) Date de publication de la demande: 29.03.2000
(73) Titulaire: L'OREAL, 75008 Paris (FR)
(72) Inventeur: PHILIPPE, Michel, F-91320 Wissous (FR); SEMERIA, Didier, F-77181 Courtry (FR)
(74) Mandataire: Dodin, Catherine
(86) Numéro de dépôt international: FR9801077
(87) Numéro de publication internationale: WO9856754

(56) Documents cités:
- WO-A-94/18940
- DE-A- 3 009 543
- GB-A- 2 001 083
- US-A- 5 476 643
- CHEMICAL ABSTRACTS, vol. 123, no. 2, 10 juillet 1995 Columbus, Ohio, US; abstract no. 19050w, J.S. PRESTON ET AL: "Solvent extraction of uranium(VI) and thorium(IV) from nitrate media by carboxylic acid amides" page 719; XP002056530 & SOLVENT EXTR. ION EXCH., vol. 13, no. 3, 1995, pages 391-413,
- M. BENOIT-GUYOD ET AL: "Recherches dans la série dipropylacétique VIII.-Structures homologues : amides et urées de la propyl-2-pentylamine" CHIMIE TH RAPEUTIQUE, vol. 7, no. 5, 1972, pages 393-398, XP002056529
- CHEMICAL ABSTRACTS, vol. 125, no. 19, 4 novembre 1996 Columbus, Ohio, US; abstract no. 247066c, T. DAGNAC ET AL: "A methodological approach to N,N-dialkylamide thermal degradation at low temperatures" page 1075; XP002056531 & J. ANAL. APPL. PYROLYSIS, vol. 37, no. 1, 1996, pages 33-47,

## Description

La présente invention concerne une nouvelle composition, notamment cosmétique, comprenant des matières pulvérulentes et des amides particuliers. L'invention concerne également l'utilisation de certains amides comme agent dispersant de matières pulvérulentes, ainsi qu'un procédé de dispersion de matières pulvérulentes.

Il est connu d'employer dans les compositions cosmétiques des matières pulvérulentes telles que des pigments ou des charges dans le but notamment de conférer à ces compositions une couleur désirée. Certains pigments d'oxydes métalliques, comme le dioxyde de titane, sont également utilisés pour leurs bonnes propriétés anti-UV connues. Toutefois, l'incorporation de ces matières pulvérulentes dans les compositions cosmétiques n'est pas toujours facile à mettre en oeuvre. En effet, on observe fréquemment l'apparition d'agglomérats et les pigments ont souvent tendance à sédimenter au cours du temps : la dispersion des pigments dans la composition n'est alors plus homogène. La sédimentation des pigments ne permet plus de conserver l'uniformité de la couleur de la composition, notamment lors de son application sur la peau. Cette sédimentation peut également engendrer une diminution sensible de l'efficacité de la protection solaire conférée par les pigments possédant une propriété anti-UV.
Pour empêcher l'agglomération et/ou la sédimentation des pigments, il a été proposé d'utiliser des agents de dispersion, et notamment des esters d'alkyle ramifiés. Par exemple, les brevets US-A-5476643 et US-A-5516506 décrivent l'emploi d'esters de néopentylglycol pour favoriser la dispersion des pigments. Selon la demande WO 94/18940, il est aussi connu d'améliorer la dispersion des pigments d'oxyde de titane en utilisant des composés organiques ramifiés tels que des esters, des éthers, des hydrocarbures ou des silicones, et en particulier le néopentanoate d'octyldodécyle.
Bien que ces agents dispersants décrits dans l'état de la technique permettent de mettre en dispersion les pigments couramment utilisés dans le domaine cosmétique, la stabilité dans le temps de ces dispersions n'est toutefois pas satisfaisante. En effet, on constate qu'après plusieurs heures de stockage, voire même plusieurs jours, la dispersion de pigments ne conserve pas son homogénéité car les pigments sédimentent au cours du temps.
Par ailleurs, il est connu par le brevet US-A-5162315 et la demande de brevet JP-A-62-215537, d'utiliser des amides comportant au moins deux chaînes alkyle pour améliorer le pénétration cutanée d'agents actifs pharmaceutiques. Dans la demande WO 88/04167, des amides comportant deux chaînes alkyle sont employés dans une composition antisolaire ou hydratante, sous forme d'émulsion, pour conférer à la composition une résistance à l'humidité.

La présente invention a pour but de permettre la préparation et l'obtention d'une composition qui comprend de matières pulvérulentes dispersées de manière homogène et qui est stable dans le temps.

La Demanderesse a découvert, de façon inattendue et surprenante, qu'en utilisant certains amides ramifiés, on pouvait obtenir une dispersion de matières pulvérulents parfaitement stable. De plus, la stabilité de la dispersion ainsi obtenue peut être conservée pendant plus d'une semaine, voire pendant plus d'un mois.

Aussi, l'invention concerne une composition comprenant au moins une matière pulvérulente et au moins un amide de formule (I) suivante :

R¹-CO-NH-R² (I)

dans laquelle R¹ et R², indépendamment l'un de l'autre, désignent un radical alkyle ramifié, saturé ou insaturé, comprenant de 3 à 30 atomes de carbone. De préférence, R¹ et R², indépendamment l'un de l'autre, désignent un radical alkyle ramifié saturé ayant de 3 à 20 atomes de carbone. Plus préférentiellement, R¹ comporte de 3 à 10 atomes de carbone et R² comporte de 10 à 20 atomes de carbone.

Avantageusement, R² désigne un radical ramifié de formule (II) : 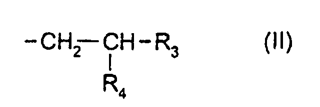 dans laquelle R₃ et R₄, indépendamment l'un de l'autre, désignent un radical alkyle linéaire comprenant de 1 à 27 atomes de carbone, sous réserve que le nombre total d'atome de carbone du radical de formule (Il) soit inférieur ou égal à 30. De préférence, R₃ et R₄, indépendamment l'un de l'autre, comportent de 2 à 12 atomes de carbone, et plus préférentiellement de 2 à 10 atomes de carbone.

Comme groupement R¹, on peut citer par exemple les groupements tert-butyle et 2,4,4-triméthyl pentyle.
Comme groupement R², on peut notamment citer les groupements 2-octyl dodécyl et 2-butyl octyl.

Parmi les composés préférés correspondant à la formule générale (I), on peut notamment citer :
- le N-néopentanoyl-2-octyl-dodécylamine,
- le N-néopentanoyl-2-butyl-octylamine,
- le N-(3,5,5-triméthyl-hexanoyl)-2-octyl-dodécylamine,
- le N-(3,5,5-triméthyl-hexanoyl)-2-butyl-octylamine.

Les composés de formule (I) sont de préférence présents en une teneur allant de 0,1% à 50 % en poids, par rapport au poids total de la composition, et mieux de 2 à 20 %.

Les matières pulvérulentes présentes dans la composition peuvent être choisies parmi les pigments, les nacres, et/ou les charges. Ils sont de préférence présents à raison de 0,1 à 80 % en poids par rapport au poids total de la composition.
Parmi les pigments, on peut citer les pigments minéraux tels que les oxydes de titane, de zinc, de fer, de zirconium, de cérium ou leurs mélanges. On peut également utiliser les nanopigments de ces oxydes métalliques qui sont connus pour leur propriété anti-UV. Ces nanopigments sont utilisés de façon connue dans les compositions anti-solaires. On entend par "nanopigments" des pigments dont la taille moyenne des particules primaires n'excède pas 100 nm, cette taille étant de préférence comprise entre 5 nm et 100 nm, et plus préférentiellement encore comprise entre 10 et 50 nm. De tels nanopigments d'oxydes métalliques, enrobés
ou non enrobés, sont des produits connus de l'homme de l'art et sont en particulier décrits dans la demande de brevet EP-A- 0 518 773, dont l'enseignement est, à cet égard, indu à titre de référence dans la présente description.
Comme pigments minéraux, on peut également citer l'oxyde de chrome, le violet de manganèse, le bleu outremer, l'hydrate de chrome et le bleu ferrique. Les pigments organiques peuvent être choisis parmi le noir de carbone, les pigments de type D & C, et les laques à base de carmin de cochenille.

Les nacres peuvent être choisies parmi les pigments nacrés blancs tels que le mica recouvert d'oxyde de titane ou d'oxychlorure de bismuth, les pigments nacrés colorés tels que le mica titane recouvert d'oxydes de fer, le mica titane avec notamment du bleu ferrique ou de l'oxyde de chrome, le mica titane avec un pigment organique du type précité ainsi que les pigments nacrés à base d'oxychlorure de bismuth.
Les charges peuvent être minérales ou organiques, lamellaires ou sphériques. On peut citer le talc, le mica, la silice, le kaolin, les poudres de Nylon, de poly-β-alanine et de polyéthylène, le Téflon, la lauroyl-lysine, l'amidon, le micatitane, la nacre naturelle, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), les oxydes de zinc et de titane, le carbonate de calcium précipité, le carbonate et l'hydrocarbonate de magnésium, l'hydroxyapatite, les microsphères de silice creuses (SILICA BEADS de MAPRECOS), les microcapsules de verre ou de céramique; les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

L'invention a également pour objet les amides choisis dans le groupe formé par :
- le N-néopentanoyl-2-octyl-dodécylamine,
- le N-néopentanoyl-2-butyl-octylamine,
- le N-(3,5,5-triméthyl-hexanoyl)-2-octyl-dodécylamine,
- le N-(3,5,5-triméthyl-hexanoyl)-2-butyl-octylamine.

Ces composés se présentent généralement sous forme de liquide huileux. On a donc constaté que la dispersion de matières pulvérulentes dans les amides selon l'invention était meilleure, plus homogène et plus stable dans le temps que la dispersion de ces mêmes matières pulvérulentes dans les huiles de l'art antérieur.

Le mélange de matières pulvérulentes et d'amides ramifiés préalablement préparé peut être introduit par exemple dans un support acceptable pour l'application envisagée, notamment dans un support cosmétiquement acceptable.

On peut encore introduire lesdits amides ramifiés et les matières pulvérulentes séparément, soit dans une composition notamment cosmétique préalablement préparée, soit directement lors du mélange de tous les constituants de la composition notamment cosmétique, selon des procédés bien connus de l'homme du métier.

La composition selon l'invention peut comprendre également au moins une huile, notamment choisie parmi les huiles végétales, animales, minérales ou synthétiques. Bien entendu, l'homme du métier veillera à employer des huiles qui ne nuisent pas à la bonne dispersion des matières pulvérulentes dans la composition, dans des quantités acceptables pour ne pas altérer ladite dispersion.

Selon une réalisation particulière de la composition de l'invention, la composition comprend comme unique huile un composé de formule (I) tel que défini précédemment.

La composition peut aussi comprendre d'autres corps gras comme les cires, qui peuvent être choisies parmi les cires animales, fossiles, végétales, minérales ou de synthèse connues en soi.

Avantageusement, la composition selon l'invention peut comprendre un support cosmétiquement acceptable.

La composition de l'invention peut également contenir au moins un additif choisi parmi les épaississants, les tensioactifs, les parfums, les conservateurs, les filtres solaires, les protéines, les vitamines, les polymères, et tout autre additif classiquement utilisé dans le domaine cosmétique. La quantité précise de chaque additif est déterminée facilement par l'homme de l'art selon sa nature et sa fonction.

Bien entendu, l'homme du métier veillera à choisir ce ou ces éventuels additifs et/ou leurs quantités de manière telle que les propriétés avantageuses, et notamment de dispersion des matières pulvérulentes, attachées intrinsèquement aux composés de formule (I) conforment à l'invention ne soient pas, ou substantiellement pas, altérées par la ou les adjonctions envisagées.

Les procédés de fabrication des compositions selon l'invention ne diffèrent en rien des procédés classiquement utilisés, notamment en cosmétique, et parfaitement connus de l'homme de l'art.

La composition selon l'invention peut se présente sous la forme d'une dispersion, d'une émulsion, notamment une émulsion eau-dans-huile ou huile-dans-eau, ou bien encore sous la forme d'une pâte souple.

Les compositions cosmétiques selon l'invention peuvent se présenter sous forme de composition de maquillage, de composition de soin de la peau, de composition capillaire ou de composition anti-solaire.

Les compositions de maquillage peuvent être sous la forme de fard à paupières, de fard à joues, d'eye-liner, de fond de teint, de blush, de mascara, de rouge à lèvres, de stick de soin des lèvres, de composition anti-cernes, de crème teintée.

Les compositions capillaires peuvent se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

L'invention concerne également l'utilisation d'un amide de formule (I) tel que défini précédemment comme agent de dispersion de matières pulvérulentes. On entend par agent de dispersion un composé apte à favoriser la dispersion desdites matières pulvérulentes.

L'invention a aussi pour objet un procédé de dispersion de matières pulvérulentes caractérisé par le fait que lesdites matières pulvérulentes sont dispersées dans une composition comprenant au moins un amide de formule (I) tel que défini précédemment.

On va maintenant donner des exemples illustrant la présente invention sans toutefois la limiter.

### Exemple 1 : Préparation du N-néopentanoyl-2-butyl-octylamine

10,4 g d'acide pivalique et 17,2 g de 2-butyl-octylamine ont été mélangés dans le tube de l'appareil micro-ondes (Maxidigest ™ MX 350 de la société PROLABO; fréquence 2450 ± 50 MHz, puissance modulable 300 W). Après une irradiation d'environ 1 heure à 160 °C ± 10 °C, le mélange réactionnel a été solubilisé dans de l'heptane puis purifié sur silice. On a obtenu 19 g de l'amide souhaité.

Le spectre RMN ¹H est conforme à la structure attendue.

| Analyse élémentaire : C₁₃ H₃₇ N O | | | | |
|---|---|---|---|---|
| | **C %** | **H %** | **N %** | **O %** |
| **Calculé** | 75,84 | 13,01 | 5,20 | 5,95 |
| **Trouvé** | 75,88 | 12,93 | 5,12 | 6,18 |

### Exemple 2 : Préparation du N-néopentanoyl-2-octyl-dodécylamine

62 ml de chlorure de pivaloyle ont été solubilisés dans 180 ml d'heptane puis additionnés à 150 g de 2-octyl-dodécylamine à une température de 60 °C. Après addition de 70 ml de triéthylamine, le milieu réactionnel a été agité pendant 2 heures, puis purifié sur silice. On a ainsi obtenu 118 g de l'amide souhaité.

Le spectre RMN ¹H est conforme à la structure attendue.

| Analyse élémentaire : C₂₆ H₄₂ N O | | | | |
|---|---|---|---|---|
| | **C%** | **H%** | **N%** | **O %** |
| **Calculé** | 78,67 | 13,47 | 3,67 | 4,19 |
| **Trouvé** | 78,54 | 13,40 | 3,63 | 4,25 |

### Exemple 3 : Préparation du N-(3,5,5-triméthyl-hexanoyl)-2-octyl-dodécylamine

3,6 g d'acide 3,5,5-triméthyl hexanoïque et 6,8 g de 2-octyl-dodécylamine ont été mélangés dans le tube de l'appareil micro-ondes (Maxidigest ™ MX 350 de la société PROLABO; fréquence 2450 ± 50 MHz, puissance modulable 300 W). Après une irradiation d'environ 50 minutes à 170 °C ± 10 °C, le mélange réactionnel a été solubilisé dans de l'heptane puis purifié sur silice. On a obtenu 7 g de l'amide souhaité.

Le spectre RMN ¹H est conforme à la structure attendue.

| Analyse élémentaire : C₂₉ H₅₉ N O | | | | |
|---|---|---|---|---|
| | **C %** | **H %** | **N%** | **O %** |
| **Calculé** | 79,63 | 13,50 | 3,20 | 3,66 |
| **Trouvé** | 79,75 | 13,32 | 3,16 | 3,93 |

### Exemple 4 : Préparation du N-(3,5,5-triméthyl-hexanoyl)-2-butyl-octylamine

29,2 g d'acide 3,5,5-triméthyl hexanoïque et 34,2 g de 2-butyl-octylamine ont été mélangés dans un récipient ouvert en pyrex placé dans un four micro-ondes (MENUMASTER ™ 3100 i; fréquence 2450, puissance à 30 % de 1400 W). Après 6 irradiations d'environ 5 minutes (pour chaque irradiation), le mélange réactionnel a été solubilisé dans de l'heptane puis purifié sur silice. On a obtenu 46 g (77 %) de l'amide souhaité.

Le spectre RMN ¹H est conforme à la structure attendue.

| Analyse élémentaire : C₂₁ H₄₂ N O | | | | |
|---|---|---|---|---|
| | **C %** | **H %** | **N %** | **O %** |
| **Calculé** | 77,54 | 13,23 | 4,31 | 4,92 |
| **Trouvé** | 77,45 | 13,06 | 4,23 | 5,12 |

### Exemple 5 : Exemples comparatifs sur les propriétés de dispersion

On a mesuré les vitesse de sédimentation d'un pigment dispersé dans différentes huiles.

### Protocole :

On a préparé un mélange de pigment et d'huile comprenant 5 % de pigment. Ce mélange a été agité pendant 20 heures à 30 °C. On a ensuite prélevé 10 ml de la dispersion obtenue après l'agitation dans un tube gradué. Puis on a mesuré le volume de pigment déposé au fond du tube (culot) en fonction du temps. On a déduit alors le volume du surnageant restant dans le tube.

Le pigment utilisé est de l'oxyde de fer rouge vendu sous la dénomination "SICOMET BRUN ZP 3569" par la société BASF.

### Résultats :

On a reporté le volume de surnageant (en ml), pour chaque huile testée, mesuré au cours du temps jusqu'à environ 200 heures.

On a obtenu les résultats suivants :
a) huiles selon l'invention :

| **Heures** | **0** | **1** | **9** | **24** | **49** | **72** | **147** | **201** |
|---|---|---|---|---|---|---|---|---|
| **Huile n° 1** | 7 | 7 | 7 | 7 | 7 | 7 | 7 | 7 |
| **Huile n° 2** | 10 | 10 | 10 | 10 | 10 | 10 | 10 | 10 |
| **Huile n° 3** | 10 | 10 | 10 | 10 | 10 | 10 | 10 | 10 |
| **Huile n° 4** | 10 | 10 | 10 | 10 | 10 | 10 | 10 | 10 |
| Huile n°1 : composé de l'exemple 1 Huile n° 2 : composé de l'exemple 2 Huile n° 3 : composé de l'exemple 3 Huile n° 4 : composé de l'exemple 4 | | | | | | | | |

On a constaté que le volume du surnageant est constant avec les 4 huiles ramifiées selon l'invention. Ces huiles permettent donc d'obtenir une dispersion stable du pigment.
b) huiles ne faisant pas partie de l'invention :

| | | | | | | | | |
|---|---|---|---|---|---|---|---|---|
| **Heures** | **0** | **6** | **12** | **25** | **50** | **100** | **150** | **200** |
| **Huile A** | 9 | 7 | 7 | 6,5 | 6,5 | 6,5 | 6,5 | 6,5 |
| **Huile B** | 10 | 9,9 | 9,8 | 9,5 | 9 | 7,5 | 4 | 3,8 |
| **Huile C** | 8 | 5,2 | 4,8 | 4,8 | 4,8 | 4,8 | 4,8 | 4,8 |
| **Huile D** | 6,5 | 4 | 3,9 | 3,8 | 3,8 | 3,8 | 3,8 | 3,8 |
| **Huile E** | -- | 9 | 6,5 | 2,75 | 2,4 | 2 | 1,9 | 1,8 |
| Huile A : Huile de Parléam Huile B : Huile de ricin Huile C : Huile de silicone (SILICONE OIL L-45 10 cst de UNION CARBIDE) Huile D : Néopentanoate d'octyle dodécyle Huile E : Huile de silicone phénylée (DOW CORNING 556 FLUID COSMETIC de DOW CORNING) | | | | | | | | |

On a constaté qu'avec les 5 huiles ne faisant pas partie de l'invention, le volume de surnageant diminue au bout de 6 heures, la diminution étant même importante après 200 heures. Les dispersions de pigment dans ces huiles ne sont donc pas stables au cours du temps, contrairement aux dispersions dans les huiles selon l'invention.

### Exemple 6 :

On a préparé une émulsion huile-dans-eau ayant la composition suivante :
- mélange d'alcool cétylstéarylique et d'alcool cétylstéarylique oxyéthyléné à 33 moles d'oxyde d'éthylène (80/20) ("DEHSCONET 390" de la société TENSIA) 7 g
- mélange de mono et distéarate de glycérol ("CERASYNTH SD" de la société ISP) 2 g
- alcool cétylique 1,5 g
- polydiméthylsiloxane ("DC200 Fluid" De DOW CORNING) 1,5 g
- composé de l'exemple 2 10 g
- Nanopigment d'oxyde de titane (MT 100 T de la société TAYCA) 5 g
- glycérine 20 g
- conservateurs qs
- eau déminéralisée qsp 100g

On a obtenu une crème fluide dans laquelle les nanopigments d'oxyde de titane sont dispersés de façon homogène dans la composition. Cette crème est utilisée comme composition anti-solaire pour le visage.

## Revendications

1. Composition comprenant au moins une matière pulvérulente **caractérisée par le fait qu'**elle comprend au moins un amide de formule (I) suivante :
R¹-CO-NH-R² (I)
dans laquelle R¹ et R², indépendamment l'un de l'autre, désignent un radical alkyle ramifié, saturé ou insaturé, comprenant de 3 à 30 atomes de carbone.

2. Composition selon la revendication 1, **caractérisée par le fait que** R¹ et R² désignent, indépendamment l'un de l'autre, un radical alkyle saturé ayant de 3 à 20 atomes de carbone.

3. Composition selon l'une des revendications précédentes, **caractérisée par le fait que** R¹ désigne un radical alkyle saturé ayant de 3 à 10 atomes de carbone.

4. Composition selon l'une des revendications 1 et 2, **caractérisée par le fait que** R² désigne un radical ramifié de formule (II) : 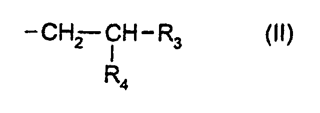 dans laquelle R₃ et R₄, indépendamment l'un de l'autre, désignent un radical alkyle linéaire comprenant de 1 à 27 atomes de carbone, sous réserve que le nombre total d'atome de carbone du radical de formule (II) soit inférieur ou égal à 30.

5. Composition selon la revendication 4, **caractérisée par le fait que** R³ et R⁴, indépendamment l'un de l'autre, comportent de 2 à 12 atomes de carbone, et de préférence, de 2 à 10 atomes de carbone.

6. Composition selon l'une quelconque des revendications précédentes, **caractérisée par le fait que** l'amide est choisi dans le groupe formé par :
- le N-néopentanoyl-2-octyl-dodécylamine,
- le N-néopentanoyl-2-butyl-octylamine,
- le N-(3,5,5-triméthyl-hexanoyl)-2-octyl-dodécylamine,
- le N-(3,5,5-triméthyl-hexanoyl)-2-butyl-octylamine.

7. Composition selon l'une quelconque des revendications précédentes, **caractérisée par le fait que** la matière pulvérulente est choisie dans le groupe formé par les pigments, les charges et les nacres.

8. Composition selon l'une quelconque des revendications précédentes, **caractérisée par le fait que** l'amide de formule (I) est présent en une teneur allant de 0,1% à 50 % en poids par rapport au poids total de la composition, et mieux de 2 à 20 %.

9. Composition selon l'une quelconque des revendications précédentes, **caractérisée par le fait que** la matière pulvérulente est présente en une teneur allant de 0,1% à 80 % en poids, par rapport au poids total de la composition.

10. Composition selon l'une quelconque des revendications précédentes, **caractérisée par le fait qu'**elle comprend un support cosmétiquement acceptable.

11. Composition cosmétique selon la revendication 10, **caractérisée par le fait qu'**elle se présente sous la forme d'une dispersion, d'une émulsion ou d'une pâte souple.

12. Composition cosmétique selon la revendication 10 ou 11, **caractérisée par le fait que** la composition se présente sous la forme d'une composition de maquillage, d'une composition de soin de la peau, d'une composition capillaire, d'une composition anti-solaire.

13. Utilisation d'un amide de formule (I) tel que défini dans l'une des revendications 1 à 6 comme agent de dispersion de matières pulvérulentes.

14. Procédé de dispersion de matières pulvérulentes **caractérisé par le fait que** lesdites matières pulvérulentes sont dispersées dans une composition comprenant au moins un amide de formule (I) tel que défini dans l'une des revendications 1 à 6.

15. Composés choisis dans le groupe formé par :
- le N-néopentanoyl-2-octyl-dodécylamine,
- le N-néopentanoyl-2-butyl-octylamine,
- le N-(3,5,5-triméthyl-hexanoyl)-2-octyl-dodécylamine,
- le N-(3,5,5-triméthyl-hexanoyl)-2-butyl-octylamine.

## Claims

1. Composition comprising at least one pulverulent material, **characterized in that** it comprises at least one amide of formula (I) below:
R¹-CO-NH-R² (I)
in which R¹ and R², independently of each other, denote a saturated or unsaturated, branched alkyl radical containing from 3 to 30 carbon atoms.

2. Composition according to Claim 1, **characterized in that** R¹ and R² denote, independently of each other, a saturated alkyl radical containing from 3 to 20 carbon atoms.

3. Composition according to either of the preceding claims, **characterized in that** R¹ denotes a saturated alkyl radical containing from 3 to 10 carbon atoms.

4. Composition according to either of Claims 1 and 2, **characterized in that** R² denotes a branched radical of formula (II) : 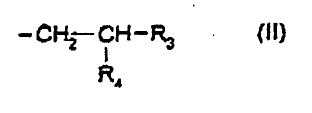 in which R₃ and R₄, independently of each other, denote a linear alkyl radical containing from 1 to 27 carbon atoms, with the proviso that the total number of carbon atoms in the radical of formula (II) is less than or equal to 30.

5. Composition according to Claim 4, **characterized in that** R₃ and R₄, independently of each other, contain from 2 to 12 carbon atoms and preferably from 2 to 10 carbon atoms.

6. Composition according to any one of the preceding claims, **characterized in that** the amide is chosen from the group formed by:
- N-neopentanoyl-2-octyldodecylamine,
- N-neopentanoyl-2-butyloctylamine,
- N- (3,5,5-trimethylhexanoyl)-2-octyldodecylamine,
- N-(3,5,5-trimethylhexanoyl)-2-butyloctylamine.

7. Composition according to any one of the. preceding claims, **characterized in that** the pulverulent material is chosen from the group formed by pigments, fillers and nacres.

8. Composition according to any one of the preceding claims, **characterized in that** the amide of formula (I) is present in a content ranging from 0.1% to 50% by weight relative to the total weight of the composition, and better still from 2 to 20%.

9. Composition according to any one of the preceding claims, **characterized in that** the pulverulent material is present in a content ranging from 0.1% to 80% by weight relative to the total weight of the composition.

10. Composition according to any one of the preceding claims, **characterized in that** it comprises a cosmetically acceptable support.

11. Cosmetic composition according to Claim 10, **characterized in that** it is in the form of a dispersion, an emulsion or a soft paste.

12. Cosmetic composition according to Claim 10 or 11, **characterized in that** the composition is in the form of a make-up composition, a skincare composition, a hair composition or an antisun composition.

13. Use of an amide of formula (I) as defined in one of Claims 1 to 6, as an agent for dispersing pulverulent materials.

14. Process for dispersing pulverulent materials, **characterized in that** the said pulverulent materials are dispersed in a composition comprising at least one amide of formula (I) as defined in one of Claims 1 to 6.

15. Compounds chosen from the group formed by:
- N-neopentanoyl-2-octyldodecylamine,
- N-neopentanoyl-2-butyloctylamine,
- N-(3,5,5-trimethylhexanoyl)-2-octyldodecylamine,
- N-(3,5,5-trimethylhexanoyl)-2-butyloctylamine.

## Patentansprüche

1. Zusammensetzung, die mindestens eine pulverförmige Substanz enthält, **dadurch gekennzeichnet, daß** sie mindestens ein Amid der folgenden Formel (I) enthält:
R¹-CO-NH-R² (I),
worin die Gruppen R¹ und R² unabhängig voneinander eine verzweigte, gesättigte oder ungesättigte Alkylgruppe mit 3 bis 30 Kohlenstoffatomen bedeuten.

2. Zusammensetzung nach Anspruch 1, **dadurch gekennzeichnet, daß** die Gruppen R¹ und R² unabhängig voneinander eine gesättigte Alkylgruppe mit 3 bis 20 Kohlenstoffatomen bedeuten.

3. Zusammensetzung nach einem der vorhergehenden Ansprüche, **dadurch gekennzeichnet, daß** R¹ eine gesättigte Alkylgruppe mit 3 bis 10 Kohlenstoffatomen ist.

4. Zusammensetzung nach einem der Ansprüche 1 und 2, **dadurch gekennzeichnet, daß** R² eine verzweigte Gruppe der Formel (II) ist: 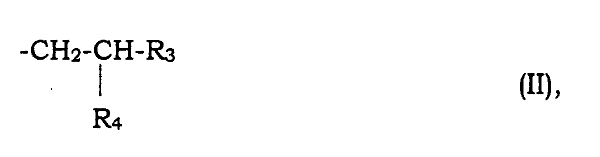 worin R₃ und R₄ unabhängig voneinander eine geradkettige Alkylgruppe mit 1 bis 27 Kohlenstoffatomen bedeuten, mit der Maßgabe, daß die Gesamtzahl der Kohlenstoffatome der Gruppe der Formel (II) höchstens 30 ist.

5. Zusammensetzung nach Anspruch 4, **dadurch gekennzeichnet, daß** die Gruppen R³ und R⁴ unabhängig voneinander 2 bis 12 Kohlenstoffatome und vorzugsweise 2 bis 10 Kohlenstoffatome enthalten.

6. Zusammensetzung nach einem der vorhergehenden Ansprüche, **dadurch gekennzeichnet, daß** das Amid ausgewählt ist unter:
- N-Neopentanoyl-2-octyldodecylamin,
- N-Neopentanoyl-2-butyloctylamin,
- N-(3,5,5-Trimethylhexanoyl)-2-octyldodecylamin,
- N-(3,5,5-Trimethylhexanoyl)-2-butyloctylamin.

7. Zusammensetzung nach einem der vorhergehenden Ansprüche, **dadurch gekennzeichnet, daß** die pulverförmige Substanz unter den Pigmenten, Füllstoffen und Perlglanzpigmenten ausgewählt ist.

8. Zusammensetzung nach einem der vorhergehenden Ansprüche, **dadurch gekennzeichnet, daß** das Amid der Formel (I) in einer Menge von 0,1 bis 50 Gew.-%, bezogen auf das Gesamtgewicht der Zusammensetzung, und besser 2 bis 20 % vorliegt.

9. Zusammensetzung nach einem der vorhergehenden Ansprüche, **dadurch gekennzeichnet, daß** die pulverförmige Substanz in einer Menge von 0,1 bis 80 Gew.-%, bezogen auf das Gesamtgewicht der Zusammensetzung, vorliegt.

10. Zusammensetzung nach einem der vorhergehenden Ansprüche, **dadurch gekennzeichnet, daß** sie einen kosmetisch akzeptablen Träger enthält.

11. Kosmetische Zusammensetzung nach Anspruch 10, **dadurch gekennzeichnet, daß** sie in Form einer Dispersion, Emulsion oder weichen Paste vorliegt.

12. Kosmetische Zusammensetzung nach Anspruch 10 oder 11, **dadurch gekennzeichnet, daß** die Zusammensetzung als Zusammensetzung zum Schminken, Zusammensetzung zur Pflege der Haut, Zusammensetzung für das Haar oder Zusammensetzung zum Sonnenschutz vorliegt.

13. Verwendung eines Amids der Formel (I) nach einem der Ansprüche 1 bis 6 als Mittel zum Dispergieren von pulverförmigen Substanzen.

14. Verfahren zum Dispergieren von pulverförmigen Substanzen, **dadurch gekennzeichnet, daß** die pulverförmigen Substanzen in einer Zusammensetzung dispergiert werden, die mindestens ein Amid der Formel (I) nach einem der Ansprüche 1 bis 6 enthält.

15. Verbindungen, die ausgewählt sind unter:
- N-Neopentanoyl-2-octyldodecylamin,
- N-Neopentanoyl-2-butyloctylamin,
- N-(3,5,5-Trimethylhexanoyl)-2-octyldodecylamin,
- N-(3,5,5-Trimethylhexanoyl)-2-butyloctylamin.
